# EUROPEAN PATENT SPECIFICATION

(11) **EP 1 814 558 B1**
(45) Date of publication and mention of the grant of the patent: **15.12.2010**
(21) Application number: 05820886.9
(22) Date of filing: 31.10.2005
(51) Int. Cl.: A61K 31/575, A61K 47/36, A61K 9/00, A61P 25/16, A61P 25/28

(54) **METHODS AND COMPOSITIONS FOR REDUCING NEURODEGENERATION IN AMYOTROPHIC LATERAL SCLEROSIS**
VERFAHREN UND ZUSAMMENSETZUNGEN ZUR VERRINGERUNG DER NEURODEGENERATION BEI AMYOTROPHISCHER LATERALSKLEROSE
PROCEDES ET COMPOSITIONS DE REDUCTION DE LA NEURODEGENERATION DANS LA SCLEROSE LATERALE AMYOTROPHIQUE

(30) Priority: 01.11.2004 US 624100 P; 16.11.2004 US 628421 P
(43) Date of publication of application: 08.08.2007
(60) Divisional application: 10176111.2
(73) Proprietor: Yoo, Seo Hong, Wyckoff, New Jersey 07481 (US)
(72) Inventor: Yoo, Seo Hong, Wyckoff, New Jersey 07481 (US)
(74) Representative: Lucas, Brian Ronald
(86) International application number: PCT/US2005/039089
(87) International publication number: WO 2006/050165

(56) References cited:
- WO-A-00/04875
- WO-A-01/56547
- WO-A-2004/012686
- KEENE C DIRK ET AL: "A bile acid protects against motor and cognitive deficits and reduces striatal degeneration in the 3-nitropropionic acid model of Huntington's disease" EXPERIMENTAL NEUROLOGY, vol. 171, no. 2, October 2001 (2001-10), pages 351-360, XP002375047 ISSN: 0014-4886
- RODRIGUES CECILIA M P ET AL: "Bilirubin and amyloid-beta peptide induce cytochrome c release through mitochondrial membrane permeabilization" MOLECULAR MEDICINE (NEW YORK), vol. 6, no. 11, November 2000 (2000-11), pages 936-946, XP009064467 ISSN: 1076-1551

## Description

The present disclosure is related to compositions and methods for ameliorating or treating at least one symptom of a neurodegenerative process or disease.

At any given time, as many as 30,000 Americans suffer with Amyotrophic Lateral Sclerosis (ALS), which is nearly always fatal. ALS, also known as Lou Gehrig's Disease, is a progressive neurodegenerative disease that attacks motor neurons in the brain and spinal cord and results in muscle weakness and atrophy. Early symptoms include loss of dexterity and gait. As the disease progresses, patients become paralyzed and require respiratory support. The life expectancy of ALS patients is usually 3 to 5 years after diagnosis with the leading cause of death being loss of respiratory function.

ALS etiology is only partially understood. Familial (inherited) cases make up only about 5-10% of ALS patients overall. Within this subset of ALS patients, one in five carry the only genetic defect identified to date, a mutation in the SODI gene. The mutant allele leads to production of a protein believed to be toxic to motor neurons. Most cases, i.e., the remaining 90-95%, arise seemingly spontaneously and without an identifiable pattern. Thus, ALS appears to be capable of striking anyone at any time. Effective therapies are scarce or non-existent.

The reader may be further enlightened as to the state of the art by reference to the following documents: WO 01/56547 A (YOO, SEO, HONG) 9 August 2001 (2001-08-09) WO 00/04875 A (YOO, SEO, HONG) 3 February 2000 (2000-02-03) WO 2004/012686 A (NITROMED, INC; GARVEY, DAVID, S; IYENGAR, RADHA) 12 February 2004 (2004-02-12) KEENE C DIRK ET AL: "A bile acid protects against motor and cognitive deficits and reduces striatal degeneration in the 3-nitropropionic acid model of Huntington's disease" EXPERIMENTAL NEUROLOGY, vol. 171, no. 2, October 2001 (2001-10), pages 351-360, XP0023750471SSN: 0014-4886 RODRIGUES CECILIA M P ET AL: "Bilirubin and amyloid-beta peptide induce cytochrome c release through mitochondrial membrane permeabilization" MOLECULAR MEDICINE (NEW YORK), vol. 6, no. 11, November 2000 (200011), pages 936-946, XP009064467 ISSN: 1076-1551. A detailed discussion of the relevance of these disclosures may be found in the prosecution file.

Accordingly the present invention provides a composition comprising a clear aqueous solution comprising Further enhancements are characterized in the dependent claims.

In embodiments containing both soluble non-starch polysaccharide and high molecular weight starch conversion product, the amounts of each are such that when combined together in the composition they are sufficient to allow the UDCA component, the high molecular weight starch conversion product, the soluble non-starch polysaccharide and the pharmaceutical compound, if any, to remain in solution at any pH within a selected pH range.

In some embodiments of the disclosure, a combination therapy composition is provided which may increase the intensity of response to or efficacy of a pharmaceutical. More specifically, administration of a composition of the disclosure comprising a UDCA and riluzole to a subject suffering from a neurodegenerative disorder may have more than an additive effect of administration of either compound alone.

Some specific example embodiments of the disclosure may be understood by referring, in part, to the following description and the accompanying drawings, wherein:
FIGURE 1A is life expectancy and its result is shown as the percent of survival on time when animal died;
FIGURE 1B is Rotarod test and its result is shown as the time they remained on the rod before sliding off on every week until dying; FIGURE 2 is a bar graph showing the results of a cell viability assay with wildtype cells, A4V cells, and G93A cells in which the cells were untreated (left panel) or incubated with 200 nM of solubilized UDCA in solution of the disclosure (center panel), or 20 µM of solubilized UDCA in solution of the disclosure (right panel);
FIGURE 3 is a bar graph showing the results of a cell viability assay with wildtype cells, A4V cells, and G93A cells in which the cells were untreated (left panel) or incubated with 500 µM S-nitrosoglutathione (GSNO; middle panel), or 500 µM GSNO followed by a 20 µM UDCA solution of the disclosure.

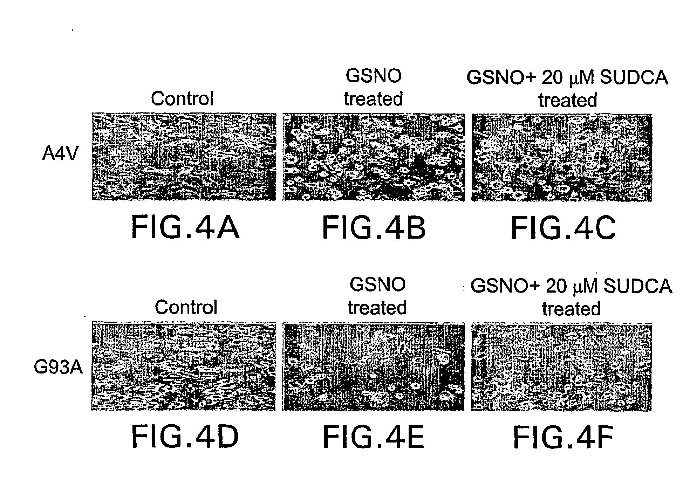

FIGURE 4A is a micrograph showing untreated A4V cells (control cells) ;
FIGURE 4B is a micrograph showing A4V cells incubated with 500 µM S-nitrosoglutathione (GSNO) ;
FIGURE 4C is a micrograph showing A4V cells incubated with 500 µM GSNO and then, were incubated in succession with 20 µM of solubilized UDCA in solution of the disclosure;
FIGURE 4D is a micrograph showing untreated G93A cells;
FIGURE 4E is a micrograph showing G93A cells incubated with 500 µM GSNO; and
FIGURE 4F is a micrograph showing G93A cells incubated with 500 µM GSNO and then, were incubated in succession with 20 µM of solubilized UDCA in solution of the disclosure.

About 85-90% of adult-onset ALS patients have no family history of the disease. This apparently random or haphazard occurrence has lead some practitioners to identify these cases as sporadic ALS (SALS). By contrast, ALS may be inherited as an autosomal dominant condition in about 10-15% of patients. These cases have been identified as familial ALS (FALS). In about a fifth of FALS cases, the mutant gene is a cytoplasmic enzyme, Cu/Zn superoxide dismutase-1 (SOD). More than 90 mutations in Cu/Zn SOD have been identified and are spread across over 30 sites. These mutations may give rise to a new adverse function that leads to FALS as opposed to simply impairing a normal function of the gene product. For example, in experiments with transgenic mice that overexpress a mutant human Cu/Zn SOD (A4V, G93A, G85R, G37R), there was no correlation between loses of SODI activity and the onset or severity of disease.

The symptoms and pathology of FALS patients with SODI mutations closely resemble those of patients with SALS. The clinical progression and pathologic alterations in -motor neurons from mice expressing mutant SODI are also strikingly similar to those found in SALS patients, suggesting that the mechanisms of neurodegeneration for SALS and FALS may share common components. Mitochondria play a pivotal role in many metabolic and apoptotic pathways that regulate the life and death of cells. Mitochondria also are the site of initiation of the intrinsic apoptotic cascade, which can be activated by the release of pro-apoptotic factors that may act both in a caspase-dependent or caspase-independent manner. Mitochondrial dysfunction, may be directly involved in the pathogenesis of ALS.

Mitochondrial dysfunction causes motor neuron death by predisposing them to calcium-mediated excitotoxicity, by increasing generation of reactive oxygen species, and/or by initiating the intrinsic apoptotic pathway. Mitochondrial dysfunction may result in quantal releases of pro-apoptotic factors, such as cytochrome c, apoptosis inducing factor (AIF), and endoG, from individual mitochondria, perhaps in response to local calcium mediated toxicity, for example, under excitatory synapses. This local toxicity might induce death of subcellular compartments, e.g., dendritic or axonal branches. This kind of subcellular compartmental degeneration might be insufficient to induce the cell to die immediately, but could spread to the cell bodies over a period of time. Consistent with this scenario, in ALS, axons degenerate from the distal to the proximal direction (dying back) and dendrites become atrophic before the final motor neuron death. This mechanism of cell death may be unique for neuronal degeneration because neurons have complex subcellular branches, and it may progress relatively slowly compared with typical cell death mechanisms in other cell types. As a consequence, it maybe that at any given time in the course of the disease, only a small number of cells are actually dying of apoptosis.

The motor neuron degeneration by SODI mutation may be investigated in cell culture and in transgenic mice models. In in vitro analyses, the spread and progressive motor neuronal death may be observed in missense mutations such as G93A (glycine to alanine at position 93) and A4V (alanine to valine at position 4) in the human Cu^{∧}Zn-superoxide dismutase gene (hSODI). For example, viability of cells with wild-type, G93A, and A4V hSODI 24 h after the neuronal differentiation was evaluated by using both the MTT assay and Trypan blue staining. Viability was significantly reduced over time. In the wild-type cells, the viability significantly decreased at 48 h after the neuronal differentiation (85.91 ± 9.08%) (P < 0.05), and was 59.41 ± 13.54% at 72 h (P < 0.01). Viability decreased even more in G93A (63.71 ± 6.25%) and A4V cells (58.85 ± 7.83%) compared with wild cells (100 + 6.97%) at 24 h after the neuronal differentiation. At 48 h, viability was further reduced to 23.12±8.96% in G93A cells and 20.79 ± 8.07% in A4V cells (P < 0.01). At 72 h, these mutant cells were nearly all dead with viabilities at about 0%. These results suggest that G93A or A4V mutations in hSODI make motor neurons more vulnerable. In view of the these results, analyses performed according to the instant disclosure may, in some embodiments, be performed at about 24 h to avoid the substantially lower viability that may occur at later time points.

The transgenic mice expressing G93A or A4V develop a severe motor neuron degenerative syndrome despite normal or above normal SOD activities. By contrast, these symptoms may not occur in mice in which Cu/Zn SOD is knocked out or overexpressed.

Nitric oxide (NO) may play a role in physiological and pathological processes in the central nervous system and mitochondria may be the primary targets for NO toxicity in the brain. NO may interact with a superoxide anion to form reactive peroxynitrites, which may cause cell death by oxidative damage, disruption of energy metabolism, calcium homeostasis, and mitochondrial function. This toxicity may be prevented by an NOS inhibitor and a nitric oxide scavenger, i.e., peroxynitrite and hydroxyl radical scavenger. S-nitrosoglutathione (GSNO) may be a useful NO donor that may slowly and spontaneously release NO under physiological conditions. GSNO may be a storage and/or transport vehicle for NO in the body. A metabolic enzyme for GSNO may be conserved from bacteria to humans. • Endogenous GSNO, which may be generated in endothelial cells and astroglial cells during oxidative stress, may be located in the cerebellum in rats. Thus, GSNO may be an endogenous NO reservoir and may play one or more roles in the brain. Interestingly, remarkable apoptosis may be observed when cells are treated with GSNO.

Some of the FALS mutant Cu'ZnSOD enzymes may induce a significantly increased peroxidative activity in comparison to the wild type protein in vitro. Peroxynitrite, a product of superoxide (O₂) and nitric oxide (NO), reacts with the Cu²⁺ of mutant SODs, producing nitronium ions, which lead to nitration of proteins and subsequent neurotoxicity, motor neurons of ALS patients exhibit increased immunoreactivity for nitrotyrosine. The enhanced peroxidase activity may increase production of hydroxyl radicals, which cold damage neurons.

In some embodiments, a bile acid composition of the disclosure may lack one or more of the disadvantageous features of existing commercial dosage forms of UDCA. In addition, a bile acid composition of the disclosure may, in some embodiments, ameliorate and/or treat at least one symptom of ALS and/or advanced ALS. Bile acid dosage forms, according to some embodiments of the disclosure, may be suitable or adaptable for oral and parenteral administration. In some embodiments, a bile acid composition of the disclosure may include an intact molecule of UDCA and an aqueous soluble starch conversion product (e.g., a product resulting from hydrolysis of starch). A bile acid composition, according to some embodiments of the disclosure, may be solubilized in water and may remain in aqueous solution without precipitation at any pH.

In some embodiments, the solubility of UDCA in a solution of the disclosure may be about 3,000 times higher than that of commercialized UDCA (0.15mol vs. 0.05mmol) and may be 300 times higher than that of TUDCA. A solution of the disclosure may, in some embodiments, deliver solubilized UDCA to blood, brain, stomach, duodenum, jejunum, ileum and/or colon. In some embodiments, an oral and parenteral dosage form may contain, for example, 500mg of UDCA and may have a Cmax that is at least 8 times higher than an existing commercial UDCA form and Tmax that is about 4-6 times shorter than an existing commercial UDCA form.

Moreover, according to some embodiments, a bile composition of the disclosure may not contain any precipitation at any pH and may function as a systemic drug. A solution, according to some embodiments, may be administered concurrently with one or more pharmaceutical compounds (e.g., a pharmaceutical compound that is therapeutically active against ALS) . Administration of a bile composition of the disclosure with another pharmaceutical compound may, in some embodiments, (a) increase the intensity of a response to the pharmaceutical compound, (b) increase the efficacy of the pharmaceutical compound, (c) decrease the required dose of the pharmaceutical compound, and/or (d> decrease the toxicity of the pharmaceutical compound. Solutions of the disclosure may also be administered separately, in terms of both the route and time of administration.

A solution of the disclosure may be used, in some embodiments, to treat or ameliorate ALS disease and/or advanced ALS disease. For example, a solution of the disclosure may include a pharmaceutical compound that decreases motor neuron death such as Pasiniazide (Tuberculostatic), Benzthiazide (Diuretic, antihypertensive), Prednisolone (Glucocorticoid), Menthol Topical analgesic, (antipuritic), Mebhydrolin Naphthalenesulfonate (Hl, antihistamine), Trichlormethiazide (Diuretic, antihypertensive), Oxytetracycline (Antibacterial), Arcaine sulphate (NOS inhibitor, NMDA inhibitor, antiprotozoal), Erythromycin (Antibacterial), Glutathione (Heavy metal poisoning, antioxidant), Trioxsalen (Melanizing agent, antipsoriatic), NylidrinHCL (Peripheral vasodilator), Desmethyldiazepam (Sedative, minor tranquilizer), Thonzylamine HCL (Antihistamine), Valproate (Na Anticonvulsant), Aminophenazone (Antipyretic, analgesic), Sulfamethizole (Antibacterial) Droperidol (Neuroleptic), 2-Thiouracil Thyroid (depressant), Kynurenic acid (Nutrient in vitamin B deficiency diseases), Fusidic acid(Antibacterial), Leucovorin Ca (Anti-anemic, antidote to folic acid antagonists), Sparteine sulfate (Oxytocic), Amygdalin (Anti-inflammatory, experimental antineoplastic) , Pratnoxine HCL (Anesthetic; topical), Furosemide (Diuretic, antihypertensive), Dinitolmide (Antiprotozoal), Budesonide (Anti-inflammatory), Flopropione (Antispasmodic), Fluorometholone (Glucocorticoid, anti-inflammatory), N-Formylmethionylphenylalanine (Chemotactic peptide), Thiopental Na (Anesthetic), Lansoprazole (Antiulcerative), Bretylium Tosylate (Inhibitor of orepinephrine release), Cefamandole Na(Antimicrobial), Oxybendazole (Antihelmintic), Cycloleucylglycine (Inhibits narcotic-induced dopamine R sensitivity), Dantrolene Na (Skeletal muscle relaxant), Tetroquinone (Keratolytic), Piperazine (Antihelmintic), Aesculin (Anti-inflammatory), Ethisterone (Progestin), Dimethadione (Anticonvulsant), Griseofulvin (Antifungal, inhibits mitosis in metaphase, interacts with, polymerized microtubules and associated proteins), Acetaminosalol (Analgesic, antipyretic), Isoguvacine HCL (GABA agonist), Putrescine DIHCL (Ornithine decarboxylase inhibitor, cell growth factor), Emetine HCL (Antiamebic, inhibits RNA, DNA, and protein synthesis), Sulfanilamide (Antibacterial), Mimosine (Depilatory agent), Acetylcholine (Cardiac depressant, miotic, peripheral vasodilator), Pralidoxime Mesylate (Cholinesterase reactivator), LysylTryptophanyl-Lysine (acetate Binds to DNA), Hecogenin (Steroid precursor), Prednisolone acetate (Glucocorticoid), Albendazole (Antihelmintic), Hydrochlorothiazide (Diuretic), Demeclocycline HCL (Antibacterial), Nitrofurazone (Topical anti-infective), Dicloxacillin Na (Antibacterial), alpha-Tocopherol (Vitamin E deficiency), Tetracycline HCL (Anti-amebic, antibacterial, antirickettsial), Fenofibrate (Antihyperlipemic), Probenecid (Uricosuric), Tretinoin (Keratolytic) Acetaminophen (Analgesic, antipyretic), Hydrastinine HCL (Cardiotonic, uterine homeostatic), d[- Arg-2] Kyotorphin acetate (Analgesic), NMDA NMDA agonist), Cefmetazole Na (Antimicrobial), Ribavirin (Antiviral), 0- Benzyl-L-Serine (Amino acid derivative), Picrotoxin (Stimulant, convulsant, GABA R antagonist, ichthyotoxin), Oxethazine (Local anesthetic), Sulfathiazole (Antibacterial) Trichlormethine (Antineoplastic, cytotoxic), Nabumetone (Anti-inflammatory), Chloramphenicol (Antibacterial, antirickettsial, inhibits protein synthesis), riluzole, ginseng and its extract, glycyrrhizin and glycyrrhizic acid, derivatives of carboquinone, coenzyme QIO, creatine, insulin-like growth factor-1, minocycline, mecamserin, xaliproden, gabapentin, dextromethorphan, talampanel, IL-I, TR-500, procysteine, brain derived neurotrophic factor, baclofen, tlzanidin, benzodiazepines, glycopyrrolate, atropine, quinine, phenytoin and morphine.

Hydrophobic bile salts fed to rats may induce apoptosis in the liver. In addition, coadministration of ursodeoxycholic acid (UDCA) may inhibit hepatocyte apoptosis in vivo. Both in hepatocytes and in nonhepatic cells apoptosis may be induced with various factors such as hydrophobic acids, ethanol, transforming growth factor-α, an agonistic Fas antibody, or okadaic acid. Surprisingly, UDCA may attenuate apoptosis and display cytoprotection by modulating mitochondrial membrane perturbation, Bax translocation and/or cytochrome c release.

Ursodeoxycholic acid (3D-7D-dihydroxy-5D-cholanic acid; UDCA) is a non-toxic hydrophilic bile acid and normally present in human bile, albeit in a low concentration of only about 3% of total bile acids. UDCA may be used for the treatment of various cholestatic disorders for which it is the only drug approved by the U.S. Food and Drug Administration (FDA). A major component of bear bile, UDCA may be useful as a pharmaceutical agent for the treatment of and the protection against many types of liver disease. Its medicinal uses at the present day include the dissolution of radiolucent - gall stones and various cholestatic disorders which are primary biliary cirrhosis, primary sclerosing cholangitis, intrahepatic cholestasis of pregnancy, cystic librosis-associated liver disease, a number of pediatric liver disorders, and chronic graft- versus-host disease of the liver.

Pharmacological action of UDCA may include replacement and/or displacement of toxic bile acids through UDCA in a dose-dependent manner, cytoprotective effects in a dose-dependent manner, stabilization/protection of cell membranes in a dose- dependent manner, antiapoptotic effects in a dose- dependent manner, immunomodulatory effects due to activation of the intracellular glucocorticoid receptor in a dose-dependent manner, antiinflammatory effects due to repression of NF-kB and inhibition of the induction of nitric oxide synthase, stimulation of bile secretion in a dose-dependent manner, Stimulation of exocytosis and insertion of canalicular membrane transporters in a dose-dependent manner.

UDCA is practically insoluble at pH 1 to 8. The solubility of its protonated form is about 0.05mM. The solubility of its taurine conjugated metabolite (UDCA; 0.45mM) is about ten times higher than UDCA solubility. Moreover, TUDCA is the only bile acid (BA) with relatively low solubility when protonated. Following oral administration, approximately 30 to 60% of UDCA is absorbed along the of the jejunum and ileum by nonionic passive diffusion and is absorbed in the ileum by active transport mechanisms and to a small extent (20% of an ingested dose) in the colon due to the insolubility of crystal UDCA, which causes extremely slow and incomplete dissolution due to the low aqueous solubility of its non-ionized molecules and more lipophilicity than the ionized bile salt species, and can therefore partition into biological membranes.

Once taken up by hepatocytes, UDCA may be conjugated to TUDCA and GUDCA, the latter two being the secreted bile acids in humans and excreted in bile by hepatic first-pass clearance. Consequently, its blood levels are extremely low in the systemic circulation. Bile acids undergo extensive hepatic recycling, or free UDCA may also be secreted by hepatocytes in bile where it may be actively and efficiently reabsorbed by cholangiocytes. UDCA and GUDCA are absorbed by both active and passive transport mechanisms, while tauro-conjugated UDCA (TUDCA) may be transported actively in the terminal ileum.

In some embodiments, a UDCA dose above 10+12 mg/kg per day may not further increase its proportion in bile since a large quantities of UDCA may be biotransformed to CDCA through 7-keto-lithocholic acid by intestinal bacteria. Alternatively, UDCA may be converted to CDCA by epimerization of the 7β-hydroxyl group and further to lithocholic acid (LCA). Therefore, with increasing doses of UDCA the absorption of UDCA decreases.

In some embodiments, administration of a composition of the disclosure may achieve adequate amounts of UDCA in the liver, in the systemic circulation, and/or in brain to have a therapeutic effect. A solution of the disclosure may, in some embodiments, display significantly increased aqueous solubility of UDCA, increased membrane permeability, protection from epimerization of UDCA to CDCA.

Survival time and quality of life for ALS patients may be improved by respiratory therapy given white sleeping during early stages of the disease and alternative feeding methods that maintain good nutrition as the disease progresses and swallowing becomes more difficult. To date, only one drug, riluzole, has been approved by the U.S. Food and Drug Administration for treatment of ALS. However, the lifespan of patients receiving riluzole is only extended by a few months. Research related to stern cells and gene therapy are promising frontiers, but have not yet enhanced the options available to treating physicians.

Without being limited to any particular mechanism of action, the present disclosure provides clear, stable solutions of soluble bile acids that may ameliorate a neurodegenerative process. In some embodiments of the disclosure, the compositions comprise riluzole. Riluzole, the only drug for treating ALS to yet receive FDA approval may function by reducing the amount of glutamate released during signal transduction. Riluzole efficacy has been demonstrated primarily in two principal controlled clinical trials. The drug's most frequent adverse events were nausea, vomiting, anorexia, diarrhea, asthenia, somnolence, vertigo, circumoral paresthesia, abdominal pain and dizziness. Of these, vertigo, diarrhea, nausea, circumoral paresthesia and anorexia appear more frequently in patients that received higher doses. Increased serum transaminase levels have generally been observed within three months of starting riluzole treatment. however, these levels recede after two to six months of treatment. Monitoring serum transaminase levels is suggested during the first year of riluzole treatment.

Bile acids may act as intracellular signaling agents, which modulate cellular transport, alter intracellular Ca2+ levels, and activate cell surface receptors. Ursodeoxycholic acid (UDCA) is a hydrophilic bile acid with proven clinical efficacy in the treatment of hepatobiliary disorders. UDCA may be rapidly conjugated with glycine or taurine in vivo to produce glycoursodeoxycolic and tauroursodeoxycholic (TUDCA) acids, respectively. UDCA and its derivatives and conjugates may function as cytoprotective agents by inhibiting apoptosis.

Since glutamate neurotoxicity may result in cell death through apoptosis, blocking apoptosis may slow acute and chronic neurodegenerative processes. In some embodiments of the disclosure, a bile composition blocks a toxic effect mediated by p53. In some embodiments of the disclosure, a bile composition blocks a toxic effects mediated by an oxidative process.

The present disclosure relates to an aqueous solution comprising (i) one or more soluble bile acids, aqueous soluble bile acid derivatives, bile acid salts, or bile acid conjugated with an amine, (collectively "bile acid"), (ii) water, and (iii) one or more aqueous soluble starch conversion products or aqueous soluble non-starch polysaccharides in an amount sufficient to produce a solution which does not form a precipitate at any pH within a desired pH range. The composition may contain a bile acid or a bile acid salt which itself has pharmaceutical effectiveness. Formulations of the disclosure may act as a carrier, an adjuvant or enhancer for the delivery of a pharmaceutical material which remains dissolved in the composition of the disclosure across the desired pH range. Alternatively, according to some embodiments of the disclosure, the composition may comprise a non-bile acid pharmaceutical that is incompletely soluble.

In some embodiments, it may be an advantage of this disclosure that the bile acid and the carbohydrate remain in solution without precipitation at any pH from acidic to alkaline. These aqueous solution systems of bile acid are substantially free of precipitate or particles. A further advantage of this disclosure is that the aqueous solution systems demonstrate no changes in physical appearance such as changes in clarity, color or odor throwing the addition of strong acids or alkali even after several months observation under accelerated conditions of storage at 50°C.

In some embodiments of the disclosure, an aqueous solution system of a bile acid is administered orally whereupon it reaches the intestine through the gastrointestinal track without precipitation of bile acids by exposure to acidic gastric juices and alkaline juices of the intestine. These dissolved bile acid formulations demonstrate intact solution systems in the intestine can be effectively and completely absorbed and, consequently, undergo enterohepatic cycling. According to an embodiment of the disclosure, bile acid solubility (e.g. precipitation and changes in physical appearance) is affected by whether a carboxylic acid side chain of certain bile acids can be protonated (non-ionized), is ionized, or is a simple carboxylic acid. The ionization state of a bile acid carboxylic acid side chain may greatly affect the hydrophobicity and the hydrophillicity of the bile acid in some aqueous solution systems. In some embodiments of the disclosure, that ionization state is manipulated by adjusting the pH to control the toxicity, absorption, and amphiphilicity of bile acids. One or more bile acids may be dissolved in these aqueous solution systems as a therapeutically active agent, as an adjuvant of a drug, as a carrier of a drug or as an enhancer of drug solubility. These aqueous solution systems may be prepared for oral consumption, enemas, mouthwashes, gargles, nasal preparations, otic preparations, injections, douches, topical skin preparations, tropical preparations, and cosmetic preparations which have a desired pH without the disadvantage of precipitation or deterioration in physical appearance after long periods of time.

Soluble bile acids are any type of aqueous soluble bile acids. A bile acid salt is any aqueous soluble salt of a bile acid. Bile salts exhibit greater solubilizing capacity for phospholipid and cholesterol and are consequently better detergents. More hydrophobic bile salts may be more injurious to various membranes, both in vivo and in vitro. Aqueous dissolved salts of bile acids may be formed by the reaction of bile acids described above and an amine including but not limited to aliphatic free amines such as trientine, diethylene triamine, tetraethylene pentamine, and basic amino acids such as arginine, lysine, ornithine, and ammonia, and amino sugars such as D-glucamine, N-alkylglucamines, and quaternary ammonium derivatives such as choline, heterocyclic amines such as piperazine, N- alkylpiperazine, piperidine, N-alkylpiperidine, morpholine, N-alkylmorphline, pyrrolidine, triethanolamine, and trimethanolamine. According to the disclosure, aqueous soluble metal salts of bile acids, inclusion compound between the bile acid and cyclodextrin and its derivatives, and aqueous soluble O-sulfonated bile acids are also included as soluble bile acid salts.

Soluble bile acid derivatives according to some embodiments of this disclosure, may be those derivatives which are as soluble in aqueous solution as or more soluble in aqueous solution than is the corresponding underivatized bile acid. Bile acid derivatives include, but are not limited to derivatives formed at the hydroxyl and carboxylic acid groups of the bile acid with other functional groups including but not limited to halogens and amino groups. Soluble bile acid may include an aqueous preparation of a free acid form of bile acid combined with one of HCl, phosphoric acid, citric acid, acetic acid, ammonia, or arginine.

Bile acids that may be used in accordance with the teachings of this disclosure include, without limitation, ursodeoxycholic acid, chenodeoxycholic acid, cholic acid, hyodeoxycholic acid, deoxycholic acid, 7-oxolithocholic acid, lithocholic acid, iododeoxycholic acid, iocholic acid, tauroursodeoxycholic acid, taurochenodeoxycholic acid, taurodeoxycholic acid, taurolithocholic acid, glycoursodeoxycholic acid, taurocholic acid, glycocholic acid, and their derivatives at a hydroxyl or carboxylic acid group on the steroid nucleus. In some embodiments of the disclosure, one advantage may be that delivery of bile acid in solution achieves higher in vivo levels of bile acids than existing commercial preparations. Therefore, the therapeutic potential of bile acid may be more fully achieved than previous formulations. The in vivo levels of bile acids attainable with existing formulations in which bile is incompletely solubilized are lower and require administration of larger amounts of bile acids. Since bile acid is completely dissolved in the inventive formulations, higher in vivo levels of bile acid may be achieved, even though lower doses are administered.

In some embodiments of the disclosure, a plurality of bile acids may be used in a single formulation. Mixtures of two or more bile salts of differing hydrophobic activity may behave as a single bile salt of an intermediate hydrophobic activity. As a result, detergent properties and the toxicity of mixtures of two bile acids of differing hydrophobic activity often are intermediate between the individual components.

Mixtures of two or more bile salts of differing hydrophobic activity may behave as a single bile salt of an intermediate hydrophobic activity. As a result, detergent properties and the toxicity of mixtures of two bile acids of differing hydrophobic activity often are intermediate between the individual components.

Carbohydrates suitable for use in the disclosure include aqueous soluble starch conversion products and aqueous soluble non-starch polysaccharides. According to some embodiments of the present disclosure, aqueous soluble starch conversion products include carbohydrates obtained directly from the partial or incomplete hydrolysis of starch under various pH conditions. Non- limiting examples include maltodextrin, dextrin, liquid glucose, corn syrup solid (dried powder of liquid glucose), and soluble starch, {e.g., maltodextrin or corn syrup solid). In some embodiments, MALTRIN^{®} M200, a corn syrup solid, and MALTRIN^{®} M700, a maltodextrin, may be used and both of which are manufactured by GPC^{®}, Grain Processing Corporation of Muscatine, Iowa may be used. For the purpose of this embodiment, the term "corn syrup" includes both corn syrup and liquid glucose. If a starch conversion product is polymeric, the polymer has at least one reducing end and at least one non-reducing end and may be linear or branched. The molecular weight may be from about 100 mass units to over 106 mass units. High molecular weight aqueous soluble starch conversion products are those having a molecular weight over 105.

According to some embodiments of the present disclosure, aqueous soluble non-starch polysaccharides may be under various pH conditions by various hydrolytic or synthetic mechanisms. Non-limiting examples include to dextran, guar gum, pectin, indigestible soluble fiber. If polymeric, the polymer has at least one reducing end and at least one non-reducing end. The polymer may be linear or branched. The molecular weight is from about 100 mass units to over 106 mass units. Preferably the molecular weight is over 105 mass units.

The amount of high molecular weight aqueous soluble starch conversion product and/or soluble non-starch polysaccharide used in embodiments of the disclosure is at least the amount needed to render the chosen bile acid(s) in the preparation soluble in the concentration desired and in the pH range desired. In some embodiments of the disclosure, the approximate minimal weight ratio- of maltodextrin to UDCA required to prevent UDCA precipitation is 6:1 (i.e. 1.2 g for every 0.2 g of UDCA, 6 g for every Ig of UDCA, and 12 g for every 2 g of UDCA in 100 mL of water) . In some embodiments of the disclosure, the approximate minimal quantity of maltodextrin is 30 g for every 200 mg of chenodeoxycholic acid, 12 g for every 200 mg of 7-ketolithocholic acid, 10 g for every 200 mg of cholic acid and 50 g for every 200 mg of deoxycholic acid. In some embodiments of the disclosure, the approximate minimal weight ratio of liquid glucose (commercial light corn syrup) to UDCA required to prevent the precipitation of bile acids from the aqueous solution dosage forms of the disclosure is about 25:1 (i.e. 12.5 g for every 500 mg UDCA in 100 mL water and 25 g for every 1 g ursodeoxycholic acid in 200 mL water). In some embodiments of the disclosure, the approximate minimal quantity of dried powder of liquid glucose (com syrup solid, e.g. MALTRIN^{®} M200) required to prevent the precipitation of bile acids from the aqueous solution dosage forms of the disclosure is 30 g for every 1 g ursodeoxycholic acid in 100 mL water, and approximately 60 g for every 2 g of ursodeoxycholic acid in 200 mL water. In some embodiments of the disclosure, the approximate minimaL quantity of soluble non-starch polysaccharide required to prevent the precipitation of bile acids from the aqueous solution dosage forms of the disclosure is 50 g guar gum for every 500 mg ursodeoxycholic acid in 100 mL water and 80g of pectin for every 500 mg of ursodeoxycholic acid in 100 mL water. The minimal required quantity of high molecular weight aqueous soluble starch conversion products or soluble non-starch polysaccharide is primarily determined by the absolute quantity of bile acids in the solution formulation rather than the concentration.

In some embodiments of the disclosure, a formulation may comprise cyclodextrin in addition to a starch conversion product and/or a non-starch polysaccharide. Alternatively, in some embodiments, a composition of the disclosure may lack cyclodextrin.

In some embodiments of the disclosure, the formulation further comprises dietary fiber. Non- limiting examples of dietary fiber include guar gum, pectin, psyllium, oat gum, soybean fiber, oat bran, corn bran, cellulose and wheat, bran.

In some embodiments of the disclosure, the formulation further comprises emulsifying agents. For the purpose of the disclosure, the term "emulsifying agent" includes emulsifying agents and suspending agents. Non-limiting examples of emulsifying agents include guar gum, pectin, acacia, carrageenan, carboxymethyl cellulose sodium, hydroxymethyl cellulose, hydroxypropyl cellulose, methyl cellulose, polyvinyl alcohol, povidone, tragacanth gum, xanthan gum, and sorbian ester.

The selected pH range for which the formulation will not precipitate its bile acid, starch conversion product, soluble non-starch polysaccharide or its pharmaceutical compound may be any range of pH levels obtainable with an aqueous system. Preferably this range is between about pH 1 and about pH 14 and more preferably between about pH 1 and about pH 10. Still more preferably the range is any subset of the range of pH levels obtainable in an aqueous system sufficient for a pharmaceutical formulation to remain in solution from preparation, to administration, to absorption in the body, according to the method of administration. Thus, the composition may be used as a pharmaceutical formulation wherein the pharmaceutical compound remains in solution without precipitation at prevailing pH levels in the mouth, stomach and intestines. In some embodiments of the disclosure, a bile acid remains dissolved under acidic conditions as a free bile acid in spite of the general insolubility of bile acids under acidic conditions.

In some embodiments of the disclosure, the pharmaceutical is riluzole. Non-limiting examples of other pharmaceutical compounds include hormones, hormone antagonists, analgesic, antipyretics, anti-inflammatory drugs, immunoactive drugs, antineoplastic drugs, antibiotics, anti-inflammatory agents, sympathomimetic drugs, anti-infective drugs, anti-tumor agents, and anesthetics. Further examples include drugs that target or affect the gastrointestinal tract, liver, cardiovascular system, and respiratory system. Further non-limiting examples of pharmaceutical compounds include insulin, heparin, calcitonin, ampicillin, octreotide, sildenafil citrate, calcitriol, dihydrotachysterol, ampomorphine, yohimbin, trazodone, acyclovir, amantadine »HCl, rimantadine»HCl, cidofovir, delavirdine*mesylate, didanosine, fameciclovir, forscarnet. sodium, fluorouracil, ganciclovir sodium, idoxuridine, interferon-α, lamivudine, nevirapine, penciclovir, ribavirin, stavudine, trifluridine, valacyclovir*HCl, zalcitabine, zidovudine, indinavir"H₂SO₄, ritonavir, netfinavir«CH₃SO₃H, saquinavir*CH₃SO₃H, d-penicillamine, chloroquine, hydroxychloroquine, aurothioglucose, gold sodium thiomalate, auranofin levamisole, DTC, isoprinosine, methyl inosine monophosphate, muramyl dipeptide, diazoxide, hydralazine»HCl, minoxidil, dipyridamole, isoxsuprine*HCl, niacin, nylidrin»HCl, phentolamine, doxazosin*CH₃S0₃H, prazosin*HCl, teraxocin»HCl, clonidine*HCl, nifedipine, molsidomine, amiodarone, acetylsalicylic acid, verapamil, diltiazem, nisoldipine, isradipine, bepridil, isosorbide»dinitrate, pentaerythrytol»tetranitrate, nitroglycerin, cimetidine, famotidine, nizatidine, ranitidine, lansoprazole, omeprazole, misoprostol, sucralfate, metoclopramide*HCl, erythromycin, bismuth compound, alprostadil, albuterol, pirbuterol, terbutaline*H₂SO₄, salmetrol, aminophylline, dyphylline, ephedrine, ethylnorepinephrine, isoetharine, isoproterenol, metaproterenol, n-docromil, oxy triphylline, theophylline, bitolterol, fenoterol, budesonide, flunisolide, beclomethasone»dipropionate, fluticasone»propionate, codeine, codeine sulfate, codeine phosphate, dextromethorphan*HBr, triamcinolone*acetonide, montelukast sodium, zafirlukast, zileuton, cromolyn sodium, ipratropium bromide, nedocromil sodium benzonate, diphenhydramine»HCl, hydrocodone*bitartarate, methadone*HCl, morphine sulfate, acetylcysteine, guaifenesin, ammonium carbonate, ammonium chloride, antimony potassium tartarate, glycerin, terpin*hydrate, colfosceril palmitate, atorvastatin*calcium, cervastatin»sodium, fluvastatin*sodium, lovastatin, pravastatin»sodium, simvastatin, picrorrhazia kurrva, andrographis paniculata, moringa oleifera, albizzia lebeck, adhata vasica, curcuma longa, momordica charantia, gymnema sylvestre, terminalia arjuna, azadirachta indica, tinosporia cordifolia, metronidazole, amphotericin B, clotrimazole, fluconazole, haloprogin, ketoconazole, griseofulvin, itraconazole, terbinafin»HCl, econazole*HN0₃, miconazole, nystatin, oxiconazole«HN0₃, sulconazole*HN0₃, cetirizine»2HCl, dexamethasone, hydrocortisone, prednisolone, cortisone, catechin and its derivatives, glycyrrhizin, glycyrrhizic acid, betamethasone, ludrocortisone»acetate, flunisolide, fluticasone»propionate, methyl prednisolone, somatostatin, lispro, glucagon, proinsulin, insoluble insulins, acarbose, chlorpropamide, glipizide, glyburide, metformin*HCl, repaglinide, tolbutamide, amino acid, colchicine, sulfinpyrazone, allopurinol, piroxicam, tolmetin sodium, indomethacin, ibuprofen, diflunisal, mefenamic acid, naproxen, and trientine.

Additional examples of pharmaceutical compounds that may be included in the formulation are any compounds which remain soluble when added to the formulation. With an additional pharmaceutical compound in the formulation, a bile acid in solution may act as an adjuvant, carrier, or enhancer for the solubility of certain therapeutically active agents, including, but not limited to, insulin (pH 7.4-7.8), heparin (pH 5-7.5), calcitonin, ampicillin, amantadine, rimantadine, sildenafil, neomycin sulfate (pH 5-7.5), apomorphine, yohimbin, trazodone, ribavirin, paclitaxel and its derivatives, retinol, and tretinoin, which are soluble and stable in acid and/or alkali and can be added as needed into these aqueous solution dosage forms of certain concentrations of bile acids in this disclosure. Certain therapeutically active agents, including, but not limited to, metformin HCl (pH 5-7), ranitidine HCl, cimetidine, lamivudine, cetrizine 2HCl (pH 4-5), amantadine, rimantadine, sildenafil, apomorphine, yohimbine, trazodone, ribavirin and dexamethasone, hydrocortisone, prednisolone, triamcinolone, cortisone, niacin, taurine, vitamins, naturally occurring amino acids, catechin and its derivatives, glycyrrhizal extract and its main constituents such as glycyrrhizin and glycyrrhizic acid, water soluble bismuth compounds (e.g., bismuth sodium tartrate), and which are soluble and stable in acid and/or alkali can be added as needed into these aqueous solution dosage formulations containing ursodeoxycholic acid in this disclosure.

Some embodiments of the disclosure may be practiced with pH adjustable agents. Non-limiting examples include HCl, H₃PO₄, H₂SO₄, HNO₃, CH₃COOH, citric acid, malic acid, tartaric acid, lactic acid, phosphate, eidetic acid and alkalies.

The disclosure contemplates treating ALS, ALS- related disorders.

In some embodiments of the disclosure, the formulation is comprised in a parenteral solution (e.g., an injectable solution, a solution, a syrup, a thick syrup or a paste. A non-limiting example of a syrup is a solution of maltodextrin wherein the concentration of maltodextrin is less than 500 g/L. A non-limiting example of a syrup is a solution of maltodextrin wherein the concentration of maltodextrin is between 500 g/L and 1.0 kg/L inclusive. A non-limiting example of a thick syrup is a solution of maltodextrin wherein the concentration of maltodextrin is between 1.0 kg/L and 1.2 kg/L inclusive. A non-limiting example of a paste is a solution of maltodextrin wherein the concentration of maltodextrin is greater than 1.2 kg/L.

The stability of dosage formulations of the disclosure may be evaluated by measuring the concentration of the relevant bile acid over time in preparations comprising soluble bite acid, a high molecular weight aqueous soluble starch conversion product, and water at various pH and temperature levels. The retention time (high performance liquid chromatography) of each bile acid may be adjusted as needed to permit individual analysis each bile acid present in complex samples, i.e. a sample having a plurality of bile acids. Stability tests may also be performed by assessing the light-scattering properties of a test solution. In addition, established accelerated testing conditions may be used.

All stability tests performed on solutions of the disclosure were satisfactory in that the concentration of bile acid as measured by HPLC did not change appreciably over time at various pH levels. Particularly, all bile acid solution formulations tested showed excellent results in the stability tests with no precipitation and no physical appearance changes over the test period. Some formulations remain stable for over 2 years. The aqueous solution dosage forms according to this disclosure that were tested did not change either physically or chemically at various pH conditions under the accelerated conditions despite the addition of therapeutically and chemically active agents that are stable and soluble in hydrochloric acid solution. Therefore, these aqueous solution systems may be extremely valuable pharmaceutical dosage forms for the therapeutically active bile acids preparations, and/or the drug (pharmaceutical compound) delivery preparations in which bile acids play roles as the adjuvant of drug, the carrier of drug, or the enhancer of solubility of a drug by micelle formation at various pH conditions without the stability problems, including precipitation in acidic conditions.

Human neuronal cells were treated with a solution of the disclosure and 50 µM of hydrogen peroxide and/or cisplatin. Hydrogen peroxide is strong oxidant. Cisplatin stimulates production of reactive oxygen species (ROS), which interfere with the antioxidant defense system. Cell cell proliferation, and apoptosis were then analyzed by measurement of MTT reduction. Several studies, using exogenous ROS, and H₂O₂, in particular, demonstrate that exposure of human and rat peripheral vascular smooth muscle cells (VSMCs) to relatively low levels of oxidant stress, for short periods promotes cell growth, whereas prolonged exposure to higher concentrations leads to cell death, either by apoptosis or necrosis.

Cell viability for hydrogen peroxide with and without solution of the disclosure was evaluated by using the MTT assay. Cells treated with a solution of the disclosure (0.2 mg/ml solubilized UDCA) and hydrogen peroxide (50 µM) displayed the highest cell viability (75% compared to control, 100%). The lowest cell viability (26% compared to control, 100%) was observed in cells treated with hydrogen peroxide (50 µM) alone. These effects were found in a dosage-dependent fashion.

Cell viability in the presence of cisplatin was evaluated in like manner. The highest cell viability (87% compared to control, 100%) was observed in cells treated with both cisplatin (20 µM) and a solution of the disclosure (1mg/ml soluble UDCA), whereas the lowest cell viability (35% compared to control, 100%) was observed in cells treated with cisplatin (20 µM) alone. These effects were also found in a dosage-dependent fashion. According to the MTT assays, a solution of the disclosure may block almost completely hydrogen peroxide-induced oxidative cytotoxicity and may completely block cisplatin-induced oxidative cytotoxicity. In conclusion, solution of the disclosure possess a strong antioxidative properties and non-cytotoxicity.

### EXAMPLES

Some embodiments of the present disclosure may be understood in connection with the following examples. However, one skilled in the art will readily appreciate the specific materials, compositions, and results described are merely illustrative of the disclosure, and are not intended to, nor should be construed to, limit the scope disclosure and its various embodiments.

### Example 1: Preparation of Bile Acid Solutions

A stock solution of bile acid was prepared by first dissolving UDCA (60 g) in 500 mL NaOH (6.7 g) solution. Next, to the resulting clear solution, 375 g of maltodextrin was added, portion by portion with vigorous agitation. The pH was then adjusted to between 7.0 and 7.2 by the dropwise addition of HCl with high throughput sonication (750W, 2OkHz). The volume was then adjusted to 1.0 L with injectable distilled water. Lastly, the resulting clear solution was filtered with sterilized using a 0.22 µ GP express plus membrane under aseptic conditions. (This filtration is important for sterilization, but not for removing paniculate matter since the solution is already clear.) Dilutions of this solution to the desired UDCA concentration were prepared according to standard pharmacy practice.

### Example 2: Preparation of Bile Acid Solutions

A stock solution of bile acid was prepared by first dissolving UDCA (25 g)- in 400 mL NaOH (2.7 g) solution. Next,- to the resulting clear solution, 745 g of maltodextrin was added, portion by portion with vigorous agitation. To this resulting solution 100 mL of a preserve solution which contains 0.95g of methyl p- hydroxybenzoate and 0.3g of sodium hydrogensulfite was added and then stirred. The volume was then adjusted to 1.0 L with pharmaceutical grade water. Lastly, the resulting, clear solution was filtered with proper filtering apparatus. (This filtration is not performed to remove particulate matter since the solution is already clear.) Dilutions of this solution to the desired UDCA concentration were prepared according to standard pharmacy practice.

### Example 3: Animal Test

**Transgenic Rat;** The transgenic animals used in this example were heteroxygotic hSOD1 carriers with a glycine93-alanine mutation (G93A). The strain is registered as B6SJL-TgN(SOD1-G93A) IGur (The Jackson Lab., Bar Harbor, ME, USA) containing a reduced copy number of hSOD1. were purchased from the Jackson Laboratories. Transgenic mice, over-expressing the human SOD1 gene with a mutation identified in ALS patients, develop an adult- onset, progressive motor deterioration. These transgenic mice are considered to be a model for the disease and have been used to test a number of strategies to delay disease progression and mortality.

In order to evaluate the potential benefit of present invention in ALS, 240mg/kg solubilized UDCA in the present solution were given by the oral administration twice per week, beginning when G93A transgenic mice were 70 days old and continuing until death.

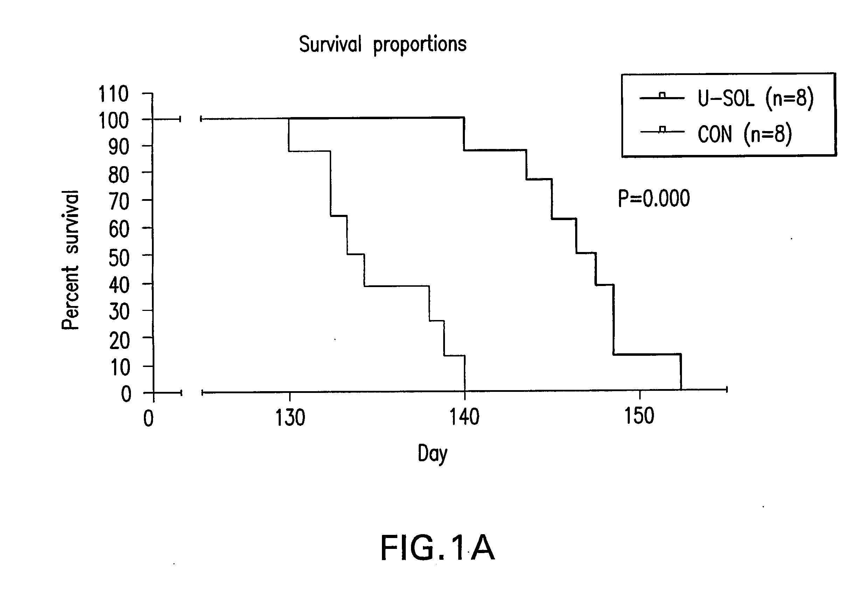

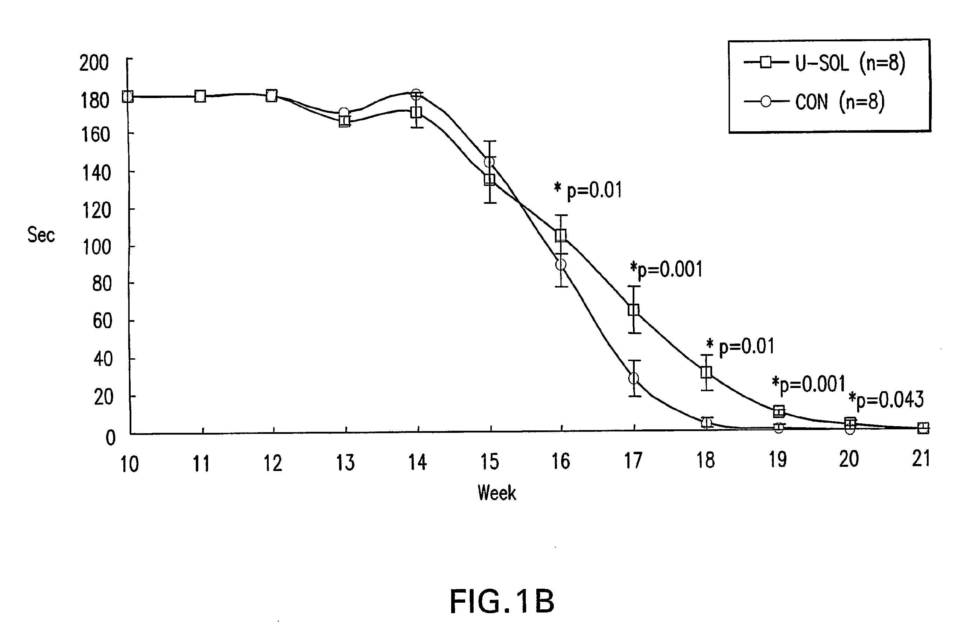

**Life expectancy and its results;** The mean life expectancy of the control mice (n=8) and the treated mice (n=8) is shown in Fig. 1. The clear solution (25 mg/kg) increased the mean survival time from 134.8 days in the control mice (n=8) to 146.6days in the treated mice (n=8), a delay of mortality with 13 days. This corresponds to- an 8.81% increase in life span of the G93A mice.

**Rotarod Test and its results;** A rotarod was used to evaluate motor performance. Mice were placed on the rod against the direction of rotation, forcing them to keep moving forwards to avoid slipping off the rod backwards. After a learning period of several days, mice were able to stay on the rotarod rotating at 15 r.p.m. Mice were tested once a week. Each mouse was given three trials on each rotarod test and the time they remained on the rod before sliding off was recorded (latency). The highest staying time on the rotarod was chosen from among the three trials as a measure for motor performance.

The motor performance of treated mice did improve greatly as the disease progressed, while the motor performance of untreated mice declined more and more rapidly as the disease progressed. Differences in motor performance between untreated and treated mice was highly significant on day 112 (p=0.01), on day 119(0.001), on day 126(p=0.01), on day 133(p=0.001) and on day 140 (p=0.043).

**Conclusion;** 70 days old, an age at which progressive cytoskeletal alterations extensively began to demonstrate in SOD1-G93A rat, has been known as clinical onset of ALS. These data demonstrate that solubilized UDCA solution significantly prolongs life span, retards the onset of paralysis and slows the evolution of functional parameters connected with muscle strength in the ALS- mouse model. Moreover, the improved motor performance on older models than 70 days old with administration of solubilized UDCA has been provided that this invent solution can treat advanced ALS disease with recovering motor performance.

### Example 4: Cell survival experiments

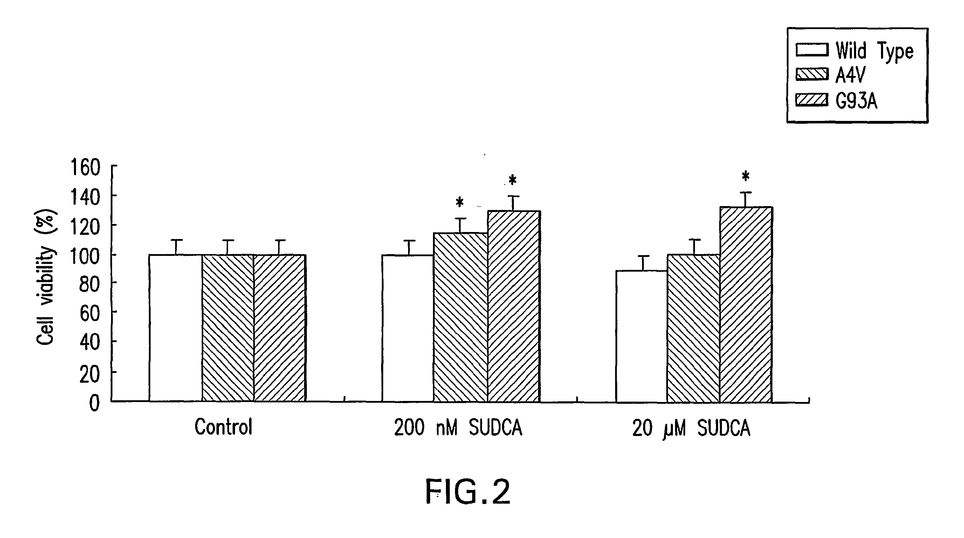

To evaluate the protective effect of solubilized UDCA solution on wild type cells, G93A cells, and A4V cells, these cells were treated with solutions containing 20OnM or 20 µM of solubilized UDCA after neuronal differentiation and then, were incubated for 24 h. The protective effect of solubilized UDCA solution on wild type cells, G93A cells, and A4V cells was determined by using both the MTT assay and Trypan blue staining. Cell viability may be expressed as a percentage of cell survival as shown in Figure 2.

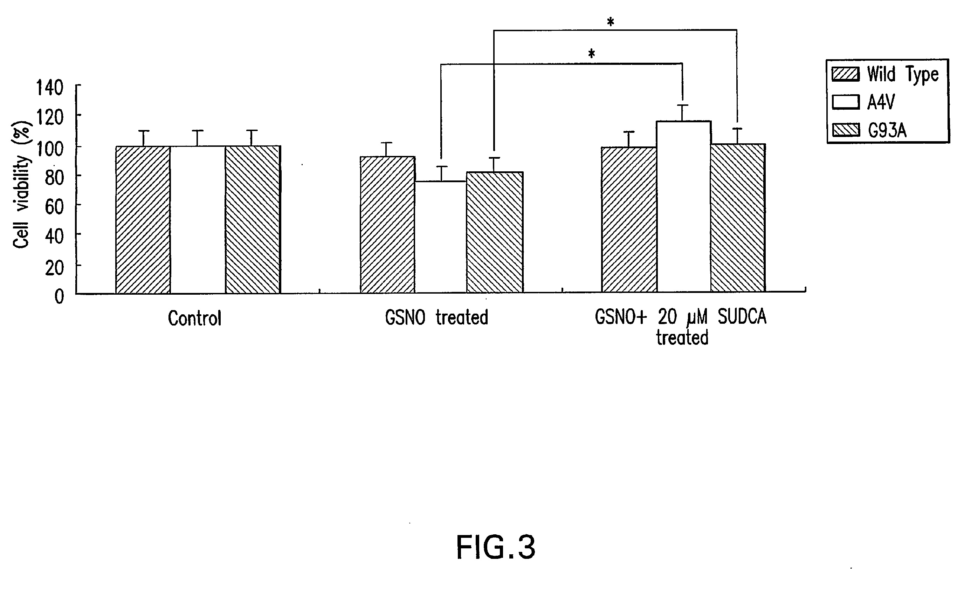

GSNO has been known to be associated with cell apoptosis. To evaluate the anti-apoptotic effect of solubilized UDCA against GSNO on wild type cells and cells containing G93A and A4V mutations in hSOD1, wildtype cells, G93A cells, and A4V cells were incubated with 500 µM GSNO for 24h in order to induce apoptosis and then cell viability of was determined. These apoptosis- induced wildtype cells, G93A cells, and A4V cells were next incubated with 20 µM of solubilized UDCA for 24h and cell viability was determined again using both the MTT assay and Trypan blue staining. Cell viability may be expressed as a percentage of cell survival as shown in Figure 3-.

**Cell culture:** VSC 4.1. (ventral spinal cord 4.1:- motoneuron-neuroblastoma hybrid cells) were maintained in Dulbecco's modified Eagles' medium/F-12 growth medium (Gibco, Grand Island, NY) with Sato's components (Sigma, St. Louis, MD) and 2% heat-inactivated newborn calf serum (HyClone, Logan, UT). They were grown in log-phase growth on poly- (L-ornithine) -precoated culture dishes (Falcon, Franklin lakes, NJ) After confluence, the cells were plated in 96-well plates (NUNC, Denmark) at a density of 1 X 10ⁿ (n=4) cells/well. For immunoblot, 1 X 10ⁿ (n=5) cells were seeded in 100 mm dishes (Falcon, Franklin Lakes, NJ).

**Constructs and establishing stable cell line:** Wild type (control) or mutant (G93A, A4V) human Cu/Zn SOD-I cDNA was cloned into the BamHI and EcoRII sites of pcDNA 3.0. An empty vector that did, not contain the insert was used for the control. Cells were tranfected (Superfect, Qiagen, Valencia, CA) and maintained in a medium that contained G418 at a concentration of 400 mg/ml (Gibco, Grand Island, NY). Single or pooled colonies were used for the experiment after clarifying the expression of human SOD1 (WT, Mutant) by Western blot analysis using an anti-human SOD1 polyclonal antibody (Calbiochem, La Jolla, CA). These cell lines were grown under the same conditions as the VSC 4.1 cells. **MTT assay and trypan blue staining to evaluate the protective effects and its results;** 3- (4,5- dimethylthiazol-2-yl) -2, 5-diphenyltetrazolium bromide (MTT) is absorbed into cells and converted to formazan by the action of mitochondrial succinate dehydrogenase. Therefore, accumulat/ion of formazan reflects the activity of mitochondria directly and the cell viability indirectly. Thus, neuronally differentiated cells (wildtype cells (control), G93A cells, A4V cells) ^{■} were plated at a density of 1 X 10ⁿ (n=4) cells^well in a 96 well plate. To evaluate the effect of G93A or A4V mutations on viability, the plated cells were incubated with the culture media containing 1 mM dibutyryl cAMP and 0.025 ug^mL aphidicolin, and the viability of wildtype cells, G93A cells, and A4V cells was evaluated as a function of concentration. To evaluate the protective effect of solubilized UDCA on viability, 20OnM and 20 µM of solubilized UDCA were added into the media. At 24 h of incubation, 50 µL of 2 mg^mL MTT (Sigma, Saint Louis, MO, USA) were added after media (200 µL) were added into each well. Subsequently, 220 µL of the solution were removed from each well, and 150 µL of dimethyl sulfoxide were then added back to each well Finally, optical density (OD) was read at 540 nm on the ELISA plate reader after particles in the well were dissolved by a microplate mixer for 10 min. Results were calibrated using OD measured without cell culture. For trypan blue staining, 10 µL of trypan blue solution were added to 10 µL of cells collected from each plate, and the cells were incubated for 2 min. Unstained live cells were counted on a haemocytometer. Cell viability was expressed as percentages of the cell survival. The viability of the wild type cells, G93A cells, and A4V cells was 100%, 130% and 115%, respectively, compared to untreated wild type cells (control; 100%) when treated with 200 nM of solubilized UDCA. At 100 times higher concentration of solubilized UDCA (20 µM) than 200 µM, the viability of wild type cells, G93A cells, and A4V cells were 89%, 133% and 101%, respectively, compared to untreated wild type cells (control; 100%). This experimental data showed that solubilized UDCA has a protective effect on G93A cells and A4V cells, and is non-toxic to wild type cells, G93A cells and A4V cells.

**DAPI staining to evaluate apoptosis by GSNO and its results;** 4',6-Diamidino-2-phenylindole dihydrochloride (DAPI) staining was performed to evaluate apoptosis as follows. After differentiation, the wildtype (control), G93A, and A4V cells were incubated with^without 500 µM GSNO for 24 h; the cells were then centrifuged at 265g for 2 min, and 4% neutral buffered formalin (100 µL) was added to the cell pellet. A 50 µL aliquot of the cell suspension was applied to a glass slide and dried at room temperature. The fixed cells were washed in PBS, air dried, and stained for 20 min with the DNA-specific fluorochrome, DAPI (Sigma, Saint Louis, MO, USA). The adhered cells were rinsed with PBS, air dried, and mounted with 90% glycerol. The slides were observed under Olympus fluorescence microscopy(pictures). Cell viability was expressed as percentages of the cell survival.

DAPI staining showed that the percent of apoptotic cells among the G93A and A4V cells (19% versus 25%, respectively) significantly increased compared with the wild cells (8%). The increased percentage of cells undergoing apoptosis in G93A and A4V cells provided evidence that apoptotic effect of GSNO was more significant on G93A cells and A4V cells than the wild type cells.

**MTT assay and trypan blue staining to evaluate the anti-apoptotic effects of solubilized UDCA and its results;** 3- (4, 5-dimethylthiazol-2-yl) -2,5-diphenyltetrazolium bromide (MTT) is absorbed into cells and converted to formazan by the action of mitochondrial succinate dehydrogenase. Therefore, accumulation of formazan reflects the activity of mitochondria directly and the cell viability indirectly. Thus, neuronally differentiated cells (wildtype cells (control), G93A cells, A4V cells) were plated at a density of 1 X 10ⁿ(n=4) cells^well in a 96 well plate. The plated cells were incubated with the culture media containing 1 mM dibutyryl cAMP and 0.025 ug^mL aphidicolin, and then, were further incubated for 24h with^without addition of 500 µM GSNO. To evaluate the anti-apoptotic effect of solubilized UDCA on cells, 20OnM and 20 µM of solubilized UDCA were added into the media. At 24 h of incubation, 50 µL of 2 mg/πiL MTT (Sigma, Saint Louis, MO, USA) were added after media (200 µL) were added into each well. Subsequently, 220 µL of the solution were removed from each well, and 150 µL of dimethyl sulfoxide were then added back to each well. Finally, optical density (OD) was read at 540 nm on the ELISA plate reader after particles in the well were dissolved by a microplate mixer for 10 min. Results were calibrated using OD measured without cell culture. For trypan blue staining, 10 µL of trypan blue solution were added to 10 µL of cells collected from each plate, and the cellls were incubated for 2 min. Unstained live cells were counted on a haemocytometer. Cell viability was expressed as percentages of the cell survival.

The decrement of viability by S-nitrosoglutathione- induced apoptosis was more prominent in G93A cells and A4V cells than in wild type cells. The increment of viability by solubilized UDCA in GSNO treated (apoptotic) cells was significant in G93A cells and A4V cells compared to the wild type cells. More specifically, compared to the wild type cells (92%), the viabilities of G93A and A4V mutations were 81% and 75%, respectively, at 24 h after the incubation with 500 µM GSNO. After adding solubilized UDCA and incubating further, the viability of the wild type cells, G93A and A4V cells increased to 98% from to 92% in the wild type cells, increased to 100% from 81% in G93A cells, and increased to 115% from to 75% in A4V cells.

This experiment demonstrates that soluble UDCA solution may protect G93A and A4V cell from NO-mediated apoptosis and may revive apoptosis-mediated damaged cells of ALS.

As will be understood by those of ordinary skill in the art, other equivalent or alternative methods, and/or compositions for ameliorating and/or treating a symptom of a neurodegenerative disease and/or a motor neuron disease. For example, methods and dosages may be scaled to diagnose and/or treat subjects of different sizes (e.g., children and adults), subjects with additional allergies or conditions, and/or subjects having varying severity of allergy and/or symptoms. In addition, methods and dosages may be adapted to fluctuations over time (e.g., monthly or seasonal). These equivalents and alternatives along with obvious changes and modifications are intended to be included within the scope of the present disclosure. For example, values and/or range endpoints provided are not intended to be rigid limits for all embodiments. Moreover, one of ordinary skill in the art will appreciate that no single embodiment, use, and/or advantage is intended to universally control or exclude other embodiments, uses, and/or advantages. For example, a medical practitioner may deem circumstances to warrant giving preference to one over another. While the present disclosure includes extensive information about current perceptions of the genetics, biochemistry, and cell biology of ALS and bile acid metabolism, future work may reveal that aspects of these perceptions are inaccurate or incomplete. Accordingly, as will be understood by those skilled in the art, the present disclosure, whether taken in whole or in part, is not limited to a particular model or mechanism of action. In addition, one of ordinary skill in the art will recognize that other equivalent or alternative compositions and methods may be used. For example, while a number of compounds have been disclosed as being capable of administration with a bile acid, other compounds may be included as well. Also, administration, of a pharmaceutical may be performed at the same time as administration of a bile acid composition or the two may simply be administered during the same or overlapping time periods (e.g., during the same hour, the same day, or the same week). Accordingly, the foregoing disclosure is intended to be illustrative, but not limiting, of the scope of the disclosure as illustrated by the following claims.

## Claims

1. A composition comprising a clear aqueous solution comprising:
(a) a first material selected from ursodeoxycholic acid or a sodium salt of ursodeoxycholic acid (UDCA);
(b) a carbohydrate selected from the group consisting of an aqueous soluble starch conversion product or an aqueous soluble non-starch polysaccharide;
(c) water, wherein the first material and the carbohydrate both remain in solution for any subset of the range of pH values obtainable in an aqueous system sufficient for a pharmaceutical formulation to remain in solution from preparation, to administration, to absorption in the body;
for use in the treatment of at least one symptom of amyotrophic lateral sclerosis.

2. The composition according to claim 1, wherein the amyotrophic lateral sclerosis is advanced amyotrophic lateral sclerosis.

3. The composition according to claim 1, wherein the symptom is selected from the group consisting of shortened lifespan and paralysis.

4. The composition according to claim 1, wherein subject is a mammal.

5. The composition according to claim 1, wherein subject is a human.

6. The composition according to claim 1, wherein the first material is present in therapeutically active amount.

7. The composition according to claim 1, wherein the aqueous soluble starch conversion product is selected from the group consisting of maltodextrin, dextrin, liquid glucose, corn syrup solid, and soluble starch.

8. The composition according to claim 1, wherein the selected pH range is between approximately 1 and approximately 10 inclusive.

9. The composition according to claim 1, wherein the aqueous soluble starch conversion product is maltodextrin.

10. The composition according to claim 1, wherein the aqueous soluble non-starch polysaccharide are selected from the group consisting of dextran, guar gum, pectin, indigestible soluble fiber.

11. The composition according to one of the preceding claims further comprising:
(d) a pharmaceutically effective amount of a pharmaceutical compound selected from one of the following:
the pharmaceutical compound is selected from the group consisting of Pasiniazide, Benzthiazide, Prednisolone, Menthol, Mebhydrolin, Naphthalenesulfonate, Trichlormethiazide, Oxytetracycline, Arcaine sulphate, Erythromycin, Glutathione, Trioxsalen, NylidrinHCL, Desmethyldiazepam, Thonzylamine HCL, Valproate Na, Aminophenazone, Sulfamethizole, Droperidol, 2-Thiouracil, Kynurenic acid, Fusidic acid, Leucovorin Ca, Sparteine sulfate, Amygdalin, Pramoxine HCL, Furosemide, Dinitolmide, Budesonide, Flopropione, Fluorometholone, anti-inflammatory), N-Formylmethionylphenylalanine, Thiopental Na, Lansoprazole, Bretylium Tosylate, Cefamandole Na, Oxybendazole, Cycloleucylglycine, Dantrolene Na, Tetroquinone, Piperazine, Aesculin, Ethisterone, Dimethadione, Griseofulvin, Acetaminosalol, Isoguvacine HCL, Putrescine DIHCL, Emetine HCL, Sulfanilamide, Mimosine, Acetylcholine, Pralidoxime Mesylate, LysylTryptophanyl-Lysine, Hecogenin, Prednisolone acetate, Albendazole, Hydrochlorothiazide, Demeclocycline HCL, Nitrofurazone, Dicloxacillin Na, alpha-Tocopherol, Tetracycline HCL, Fenofibrate, Probenecid, Tretinoin, Acetaminophen, Hydrastinine HCL, d[-Arg-2]Kyotorphin acetate, NMDA, Cefmetazole Na, Ribavirin, Benzyl-L-Serine, Picrotoxin, Oxethazine, Sulfathiazole, Trichlormethine, Nabumetone, Chloramphenicol, riluzole, ginseng and its extract, glycyrrhizin and glycyrrhizic acid, derivatives of carboquinone, coenzyme Q10, creatine, insulin-like growth factor-I, minocycline, mecamserin, xaliproden, gabapentin, dextromethorphan, talampanel, IL-1, TR500, procysteine, brain derived neurotrophic factor, baclofen, tizanidin, benzodiazepines, glycopyrrolate, atropine, quinine, phenytoin and morphine
that decreases motor neuron death, and
wherein the first material, the carbohydrate, and the pharmaceutically effective amount of the pharmaceutical compound all remain in solution for all pH values of the solution within a selected range of pH values.

12. The composition according to claim 11, wherein the first material is present in a neuroprotective amount.

## Patentansprüche

1. Zusammensetzung, umfassend eine klare wässrige Lösung, umfassend:
(a) ein erstes Material ausgewählt aus Ursodeoxycholsäure oder einem Natriumsalz der Ursodeoxycholsäure (UDCA);
(b) ein Kohlenhydrat, ausgewählt aus der Gruppe bestehend aus einem wässrigen, löslichen Stärkeumsetzungsprodukt oder einem wässrigen, löslichen Nicht-Stärke-Polysaccharid;
(c) Wasser, wobei sowohl das erste Material und das Kohlenhydrat für eine Teilmenge des pH-Wertbereichs in einem wässrigen System in Lösung verbleibt, das für eine pharmazeutische Formulierung ausreichend ist, um von Herstellung zur Verabreichung bis hin zur Absorption in den Körper in Lösung zu verbleiben;
zur Anwendung bei der Behandlung wenigstens eines der Symptome der amyothrophen Lateralsklerose.

2. Zusammensetzung nach Anspruch 1, wobei es sich bei der amyotrophen Lateralsklerose um eine fortgeschrittene amyotrophe Lateralsklerose handelt.

3. Zusammensetzung nach Anspruch 1, wobei das Symptom ausgewählt ist aus der Gruppe bestehend aus einer verkürzten Lebensspanne sowie Paralyse.

4. Zusammensetzung nach Anspruch 1, wobei es sich bei der Testperson um ein Säugetier handelt.

5. Zusammensetzung nach Anspruch 1, wobei es sich bei der Testperson um einen Menschen handelt.

6. Zusammensetzung nach Anspruch 1, wobei das erste Material in einer therapeutisch aktiven Menge anwesend ist.

7. Zusammensetzung nach Anspruch 1, wobei das wässrige lösliche Stärkeumsetzungsprodukt ausgewählt ist aus der Gruppe bestehend aus Maltodextrin, Dextrin, flüssiger Glucose, Maissyrup-Feststoff und löslicher Stärke.

8. Zusammensetzung nach Anspruch 1, wobei der ausgewählte pH-Bereich zwischen ungefähr 1 und ungefähr einschließlich 10 beträgt.

9. Zusammensetzung nach Anspruch 1, wobei das wässrige lösliche Stärkeumsetzungsprodukt Maltodextrin ist.

10. Zusammensetzung nach Anspruch 1, wobei das wässrige lösliche Nicht-Stärke-Polyaccharid ausgewählt ist aus der Gruppe bestehend aus Dextran, Guargummi, Pektin, unverdaulichem löslichem Ballaststoff.

11. Zusammensetzung nach einem der vorstehenden Ansprüche, ferner umfassend:
(d) eine pharmazeutisch wirksame Menge einer pharmazeutischen Verbindung, ausgewählt aus dem Folgenden:
die pharmazeutische Verbindung ist ausgewählt aus der Gruppe bestehend aus Pasiniazid, Benzthiazid, Prednisolon, Menthol, Mebhydrolin, Naphthalensulfonat, Trichlormethiazid, Oxytetracyclin, Arcaine-Sulfat, Erythromycin, Glutathion, Trioxsalen, Nylidrin-HCL, Desmethyldiazepan, Thonzylamin-HCL, Vaproat-Na, Aminophenazon, Sulfamethiozol, Droperidol, 2-Thiouracil, Kynurensäure, Fusidinsäure, Leucovorin-Ca, Sparteinsulfat, Amygdalin, Pramoxin-HCL, Furosemid, Dinitolmid, Budesonid, Fopropion, Fluormetholon, entzündungshemmend), N-Formylmethionylphenylalanin, Thiopental Na, Lansoprazol, Bretyliumtosylat, Cefamandol Na, Oxybendazol, Cycloleucylglycin, Dantrolen Na, Tetroquinon, Piperazin, Aesculin, Ethisteron, Dimethadion, Griseofulvin, Acetaminosalol, Isoguvacin-Hcl, Putrescin-DIHCL, Emetin-HCL, Sulfanilamid, Mimosin, Acetylcholin, Pralidoximmesylat, Lysyltryptophanyl-Lysin, Hecogenin, Prednisolonacetat, Albendazol, Hydrochlorthiazid, Demecloczclin-HCL, Nitrofurazon, Dicloxacillin Na, Alpha-Tocopherol, Tetracyclin-HCL, Fenofibrat, Probenecid, Tretinoin, Acetaminophen, Hydrastinin-HCL, d[-Arg-2]Kyotrophinacetat, NMDA, Cefmetazol Na, Ribavirin, Benzyl-L-Serin, Picrotoxin, Oxethazin, Sulfathiazol, Trichlormethin, Nabumeton, Chloramphenicol, Riluzol, Ginseng und dessen Extrakte, Glycyrrhizin sowie Glycyrrhizinsäure, Derivate von Carboquinon, Coenzym Q10, Kreatin, insulinähnlichem Wachstumsfaktor I, Minocyclin, Mecamserin, Xaliproden, Gabapentin, Dextromethorphan, Talampanel, IL-1, TR500, Procystein, vom Gehim abgeleitetem neurotrophischem Faktor, Baclofen, Tiyanidin, Benzodiazepine, Glycopyrrolat, Atropin, Quinin, Phenytoin und Morphin,
welche den motorischen Nervenzellentod reduzieren, und
wobei das erste Material, das Kohlenhydrat und die pharmazeutisch wirksame Menge der pharmazeutischen Verbindung jeweils für sämtliche pH-Werte der Lösung innerhalb eines ausgewählten pH-Wertbereichs in Lösung verbleiben.

12. Zusammensetzung nach Anspruch 11, wobei das erste Material in einer neuroprotektiven Menge vorliegt.

## Revendications

1. Composition comportant une solution aqueuse limpide comprenant :
(a) un premier matériau sélectionné parmi l'acide ursodésoxycholique ou un sel de sodium d'acide ursodésoxycholique (AUDC) ;
(b) un hydrate de carbone sélectionné dans le groupe constitué d'un produit de conversion d'amidon aqueux soluble ou d'un polysaccharide non-amidon aqueux soluble ;
(c) de l'eau, dans laquelle le premier matériau et l'hydrate de carbone restent tous les deux en solution pour n'importe quel sous-ensemble de la plage des valeurs de pH pouvant être obtenues dans un système aqueux suffisant pour qu'une formulation pharmaceutique reste en solution durant la préparation, l'administration et l'absorption dans le corps ;
à utiliser dans le traitement d'au moins un symptôme de la sclérose latérale amyotrophique.

2. Composition selon la revendication 1, dans laquelle ladite sclérose latérale amyotrophique est une sclérose latérale amyotrophique avancée.

3. Composition selon la revendication 1, dans laquelle le symptôme est sélectionné dans le groupe constitué d'une réduction de la durée de vie et de paralysie.

4. Composition selon la revendication 1, dans laquelle le sujet est un mammifère.

5. Composition selon la revendication 1, dans laquelle le sujet est un être humain.

6. Composition selon la revendication 1, dans laquelle le premier matériau est présent en quantité thérapeutiquement active.

7. Composition selon la revendication 1, dans laquelle le produit de conversion d'amidon aqueux soluble est sélectionné dans le groupe constitué de maltodextrine, de dextrine, de sirop de glucose, de sirop de glucose déshydraté et d'amidon soluble.

8. Composition selon la revendication 1, dans laquelle la plage de pH sélectionnée est comprise entre environ 1 et environ 10 inclus.

9. Composition selon la revendication 1, dans laquelle le produit de conversion d'amidon aqueux soluble est la maltodextrine.

10. Composition selon la revendication 1, dans laquelle le polysaccharide non-amidon aqueux soluble est sélectionné dans le groupe constitué de dextrane, de gomme de guar, de pectine et de fibre soluble non digestible.

11. Composition selon l'une des revendications précédentes comprenant en outre :
(d) une quantité pharmaceutiquement efficace d'un composé pharmaceutique sélectionné parmi l'un des éléments suivants :
Le composé pharmaceutique est sélectionné dans le groupe constitué de pasiniazide, de benzthiazide, de prednisolone, de menthol, de mébhydroline, de sulfonate de naphtalène, de trichlorméthiazide, d'oxytétracycline, de sulfate d'arcaïne, d'érythromycine, de glutathion, de trioxsalène, de chlorhydrate de nylidrine, de desméthyldiazépam, de chlorhydrate de thonzylamine, de valproate de sodium, d'aminophénazone, de sulfaméthizol, de dropéridol, de 2-thiouracile, d'acide kynurénique, d'acide fusidique, de leucovorine calcique, de sulfate de spartéine, d'amygdaline, de chlorhydrate de pramoxine, de furosémide, de dinitolmide, de budésonide, de flopropione, de fluorométholone, d'anti-inflammatoire, de N-formyl-méthionyl-phénylalanine, de thiopental sodique, de lansoprazole, de tosylate de brétylium, de céfamandole sodique, d'oxybendazole, de cycloleucylglycine, de dantrolène sodique, de tétroquinone, de pipérazine, d'esculine, d'éthistérone, de diméthadione, de griséofulvine, d'acétaminosalol, de chlorhydrate d'isoguvacine, de dichlorhydrate de putrescine, de chlorhydrate d'émétine, de sulfanilamide, de mimosine, d'acétylcholine, de mésylate de pralidoxime, de lysyltryptophanyl-lysine, d'hécogénine, d'acétate de prednisolone, d'albendazole, d'hydrochlorothiazide, de chlorhydrate de déméclocycline, de nitrofurazone, de dicloxacilline sodique, d'alpha-tocophérol, de chlorhydrate de tétracycline, de fénofibrate, de probénécide, de trétinoïne, d'acétaminophène, de chlorhydrate d'hydrastinine, d'acétate de d[-Arg-2]kyotorphine, de NMDA, de cefmétazole sodique, de ribavirine, de benzyl-L-sérine, de picrotoxin, d'oxéthazine, de sulfathiazol, de trichlorméthine, de nabumétone, de chloramphénicol, de riluzole, de ginseng et de son extrait, de glycyrrhizine et d'acide glycyrrhizique, de dérivés de carboquinone, de coenzyme Q10, de créatine, de facteur de croissance insulinomimétique de type I, de minocycline, de mecamserin, de xaliprodène, de gabapentine, de dextrométhorphane, de talampanel, d'IL-1, de TR500, de procystéine, de facteur neurotrophique dérivé du cerveau, de baclofène, de tizanidine, de benzodiazépines, de glycopyrrolate, d'atropine, de quinine, de phénytoïne et de morphine
qui diminue la mort des neurones moteurs, et
dans laquelle le premier matériau, l'hydrate de carbone et la quantité pharmaceutiquement efficace du composé pharmaceutique restent tous en solution pour toutes les valeurs de pH de la solution dans une plage sélectionnée des valeurs de pH.

12. Composition selon la revendication 11, dans laquelle le premier matériau est présent en une quantité neuroprotectrice.
